# EUROPEAN PATENT SPECIFICATION

(11) **EP 4 327 863 B1**
(45) Date of publication and mention of the grant of the patent: **15.10.2025**
(21) Application number: 22791918.0
(22) Date of filing: 29.03.2022
(51) Int. Cl.: A61N 5/06, A61N 5/067

(54) **LOW-LEVEL LASER THERAPY DEVICE USING LED ENDOSCOPE CAP**
LOW-LEVEL-LASERTHERAPIEVORRICHTUNG MIT LED-ENDOSKOPKAPPE
DISPOSITIF DE THÉRAPIE LASER DE FAIBLE NIVEAU UTILISANT UN CAPUCHON D'ENDOSCOPE À LED

(30) Priority: 22.04.2021 KR 20210052572
(43) Date of publication of application: 28.02.2024
(73) Proprietor: The Asan Foundation, Seoul 05505 (KR); University Of Ulsan Foundation For Industry Cooperation, Ulsan 44610 (KR)
(72) Inventor: PARK, Do Hyun, Seoul 05555 (KR); KWON, Jin Hee, Seoul 05544 (KR)
(74) Representative: AWA Sweden AB
(86) International application number: PCT/KR2022/004465
(87) International publication number: WO 2022/225218

(56) References cited:
- EP-A1- 3 701 850
- WO-A1-2016/047191
- JP-A- 2012 239 833
- JP-A- 2013 031 541
- JP-A- 2019 150 388
- KR-A- 20160 104 445
- KR-B1- 101 971 185

## Description

### TECHNICAL FIELD

The present disclosure relates to a low-level laser therapy device using a light-emitting diode (LED) endoscope cap.

### BACKGROUND ART

The duodenum is a site of increased secretion of type 2 diabetes/non-alcoholic fatty liver-related hormones (e.g., glucose-dependent insulinotropic polypeptide (GIP)) as abnormal mucosal thickening occurs in patients with type 2 diabetes, and is a key location for non-drug therapy for type 2 diabetes, and obesity and non-alcoholic fatty liver.

GIP is secreted by K-cells in the duodenum and pancreas, and stimulation of the K-cells with phototherapy improves intracellular mitochondrial dysfunction and thereby regulates GIP secretion, which may prevent type 2 diabetes and obesity and non-alcoholic fatty liver.

A duodenal mucosal resurfacing procedure using a high frequency or the like may be applied as one of the therapy procedures for such diseases. However, there is a problem that such high-frequency therapy using heat may cause narrowing, perforation, and bleeding of the duodenum. On the other hand, a phototherapy method using a low-level laser may treat diabetes by irradiating the duodenal surface with light to improve the mitochondrial function of abnormal cells, inhibit K-cell function by increasing adenosine triphosphate (ATP) production, regenerate normal duodenal and pancreatic cells, and improve the environment of intestinal bacteria in the duodenum.

Meanwhile, the phototherapy method should be performed by inserting a catheter such as an endoscope into the patient's body, and thus, during the process thereof, there is a possibility that the catheter or a cap attached to the outer side of the catheter may damage the organs, the mucous membranes, or the like inside the patient's body.

In addition, for efficient therapy, it is preferable that a light source such as a light-emitting diode (LED) is in contact with a surface of the duodenum or the like as widely as possible.

Accordingly, there is a need to develop a therapy device that allows a light source to come into contact with the surface of the internal organ without damaging the patient's organs or mucous membranes with a catheter or the like inserted into the patient's body.

Document KR20160104445A discloses a photodynamic therapy cap which is coupled to the tip of an endoscope and comprises expandable light irradiation units.

### DESCRIPTION OF EMBODIMENTS

### TECHNICAL PROBLEM

The present disclosure is directed to providing a laser therapy device that allows a light source to come into contact with the surface of the internal organ without damaging the surface of the patient's organ, mucous membranes, or the like.

The present disclosure is also directed to providing a laser therapy device capable of irradiating light of different wavelengths to the patient's duodenum and pancreas.

However, these objects are exemplary, and the scope of the present disclosure is not limited thereto.

### TECHNICAL SOLUTION TO PROBLEM

The invention is defined in the appended set of claims. In order to achieve the above-described purpose, a low-level laser therapy device according to the invention includes a catheter formed to extend so as to be inserted into a body of a patient and including an air insufflation part for stretching the mucosal folds of the duodenum of the patient, an endoscope cap having a through hole therein to allow the catheter to be inserted therethrough, a hood disposed in one region outside the endoscope cap and disposed such that at least one region thereof protrudes forward from the endoscope cap, and a plurality of LEDs disposed on each of the endoscope cap and the hood, wherein a plurality of first LEDs is disposed on a surface of the endoscope cap to form a plurality of columns and plurality of rows, wherein a plurality of second LEDs is disposed on a surface of the hood to form a plurality of columns and plurality of rows, wherein the hood is formed to have a joint structure and is movable such that at least one region thereof forms a predetermined angle with respect to the endoscope cap.

### ADVANTAGEOUS EFFECTS OF DISCLOSURE

The present disclosure can provide a laser therapy device that allows a light source to come into contact with the surface of the internal organ without damaging the surface of the patient's organ, mucous membranes, or the like.

In addition, the present disclosure can provide a laser therapy device capable of irradiating light of different wavelengths to the patient's duodenum and pancreas.

However, these effects are exemplary and do not limit the present disclosure.

### BRIEF DESCRIPTION OF DRAWINGS

FIG. 1 is a diagram illustrating the results of experiments comparing blood glucose levels in diabetic mice with and without providing light-emitting diode (LED) therapy.
FIG. 2 is a view schematically illustrating a low-level laser therapy device using an LED endoscope cap according to an embodiment of the present disclosure.
FIG. 3 is a view schematically illustrating an embodiment of a body of FIG. 2.
FIG. 4A is a bottom view of the body of FIG. 2, and FIG. 4B is a top view of the body of FIG. 2.
FIG. 5 is a diagram for describing light emitted by LEDs.
FIG. 6 is a view for describing an example of use of the low-level laser therapy device using an LED endoscope cap according to the present disclosure.
FIG. 7 is an enlarged view of portion x of FIG. 3.
FIG. 8 is a side view of the low-level laser therapy device using an LED endoscope cap of FIG. 2.
FIG. 9 is a view illustrating an embodiment of a hood of FIG. 3.
FIG. 10 is a view illustrating another embodiment of the hood of FIG. 3.
FIG. 11 is a view for describing the interior of the body.

In order to achieve the above-described purpose, the low-level therapy device includes a catheter formed to extend so as to be inserted into a body of a patient and including an air insufflation part for stretching the mucosal folds of the duodenum of the patient, an endoscope cap having a through hole therein to allow the catheter to be inserted therethrough, a hood disposed in one region outside the endoscope cap and disposed such that at least one region thereof protrudes forward from the endoscope cap, and a plurality of light-emitting diode (LED)s disposed on each of the endoscope cap and the hood. A plurality of first LEDs is disposed on a surface of the endoscope cap to form a plurality of columns and plurality of rows, a plurality of second LEDs is disposed on a surface of the hood to form a plurality of columns and plurality of rows.

In addition, the hood is formed as a joint structure.

In addition, the hood may include a curved region formed as a curved surface at one end thereof.

In addition, a first LED configured to irradiate a first light of a first wavelength band may be disposed on a surface of the endoscope cap, and a second LED configured to irradiate a second light of a second wavelength band different from the first wavelength band may be disposed on a surface of the hood.

In addition, the first wavelength band may be a predetermined wavelength band between 600 nm and 700 nm, and the second wavelength band may be a predetermined wavelength band between 800 nm and 900 nm.

In addition, the hood is movable such that at least one region thereof forms a predetermined angle with respect to the endoscope cap.

In addition, a band having a diameter less than a diameter of the through hole may be inserted into the through hole.

In addition, the LEDs may include at least one of a quantum dot LED with quantum dots as a light source, and a flexible organic light-emitting diode (OLED).

### MODE OF DISCLOSURE

While the present disclosure is susceptible to various modifications and alternative forms, specific embodiments thereof are shown by way of example in the drawings and will herein be described in detail. Advantages and features of the present disclosure and methods for accomplishing the same will be more clearly understood from embodiments described below with reference to the drawings. However, the present disclosure is not limited to the embodiments disclosed below but may be implemented in various forms.

Hereinafter, the embodiments of the present disclosure will be described below in detail with reference to the accompanying drawings, but when describing with reference to the drawings, equal or corresponding components will be referred to as the same reference numerals, and redundant descriptions thereof will be omitted.

In the following embodiments, the terms "first", "second", and the like have been used to distinguish one component from another, rather than limitative in all aspects.

In the following embodiments, singular forms are intended to include plural forms as well, unless the context clearly indicates otherwise.

It will be further understood that the terms "including" and/or "having" used herein specify the presence of stated features or components, but do not preclude the presence or addition of one or more other features or components.

Sizes of components in the drawings may be exaggerated or reduced for convenience of description. For example, the size and thickness of each component shown in the drawings are arbitrarily represented for convenience of description, and thus, the present disclosure is not necessarily limited thereto.

In the following embodiments, an x-axis, a y-axis, and a z-axis are not limited to three axes of the rectangular coordinate system, and may be interpreted in a broader sense. For example, the x-axis, the y-axis, and the z-axis may be perpendicular to one another, or may represent different directions that are not perpendicular to one another.

When a certain embodiment may be implemented differently, a specific process order may be performed differently from the described order. For example, two consecutively described processes may be performed substantially at the same time or performed in an order opposite to the described order.

Hereinafter, a low-level laser therapy device 10 using a light-emitting diode (LED) endoscope cap according to the present disclosure will be described in detail in accordance with the foregoing principles.

There is a problem that intestinal bacteria harmful to the human body may lead to a reduction in short-chain fatty acids in the body, thereby damaging the duodenal mucosa, and this damage may cause metabolites produced by the intestinal bacteria harmful to the human body to spread throughout the body via the bloodstream, leading to various diseases such as metabolic diseases.

In order to address this problem, the present disclosure can provide the function of photobiomodulation therapy via LEDs. For example, according to the present disclosure, by photobiomodulation of the duodenal microbiome, the growth of intestinal bacteria (such as Clostridium) that are harmful to the human body may be suppressed and beneficial intestinal bacteria (such as Bifidobacterium and Lactobacillus) may be photo microbiota transplanted (PMT)) in the duodenum. Accordingly, the present disclosure has the effect of treating metabolic diseases including type 2 diabetes and fatty liver, and improving neurological diseases, such as dementia caused by the imbalance of intestinal bacteria in the duodenal microbiome, and heart and cancer diseases, by improving duodenal mucosa and intestinal bacterial environment.

As a result, the present disclosure has the effect of reducing harmful bacteria and increasing beneficial bacteria in the patient's duodenum through PMT.

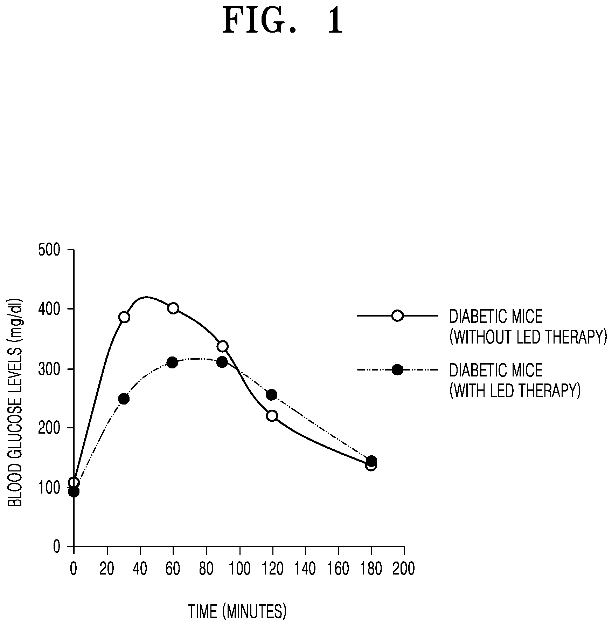

FIG. 1 is a diagram illustrating the results of experiments comparing blood glucose levels in diabetic mice with and without providing LED therapy.

According to the results of experiments on diabetic model mice using the low-level laser therapy device 10 (hereinafter referred to as a laser therapy device) using an LED endoscope cap according to the present disclosure illustrated in FIG. 1, the results indicate that LED therapy may lead to a reduction in blood glucose levels solely through light irradiation, without generating any heating effects.

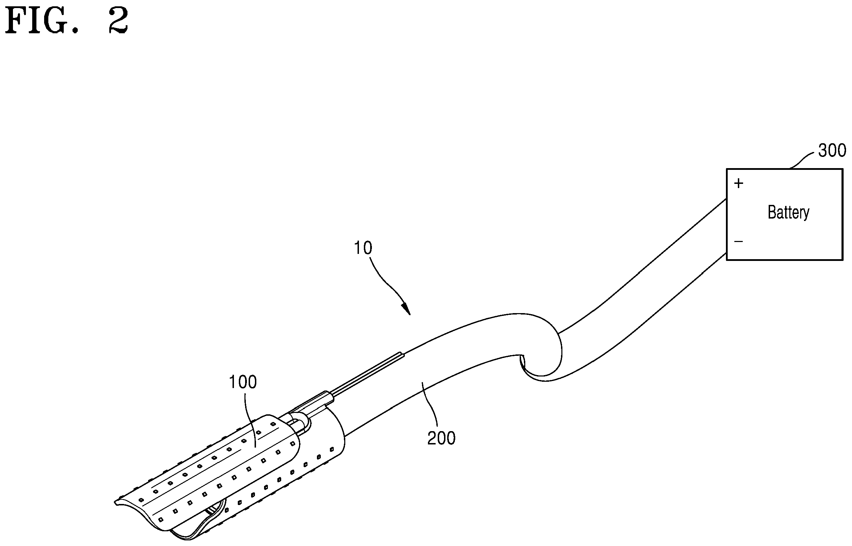

FIG. 2 is a view schematically illustrating the laser therapy device 10 according to an embodiment of the present disclosure.

Referring to FIG. 2, the laser therapy device 10 according to an embodiment of the present disclosure may include a body 100, a catheter 200, and a battery 300.

The catheter 200 may be a component inserted into a patient's body. For example, the catheter 200 may be an endoscope tube formed of a flexible material.

The catheter 200 may be in the shape of a tube having a circular cross-section. Alternatively, the catheter 200 may be in the shape of a tube having a polygonal cross-section.

An endoscope cap 110 may be connected to one side of the catheter 200 as will be described below. For example, the catheter 200 may be inserted into a through hole 111 formed in the endoscope cap 110.

The catheter 200 may guide the movement of the endoscope cap 110 while being inserted into the patient's body. That is, when the catheter 200 is inserted into the patient, the endoscope cap 110 connected to one side of the catheter 200 moves together therewith, thereby guiding the path inside the patient's body, through which the endoscope cap 110 moves. For example, the catheter 200 may be inserted into the duodenum of a user.

One end of the laser therapy device according to the present embodiment may be connected to the battery 300. The battery 300 may be a medical battery, and the laser therapy device 10 may receive power for laser therapy therefrom.

In addition, the laser therapy device 10 may receive power required for light irradiation from a medical high-frequency generator or a medical low-power generator in the form coupled to an extracorporeal end.

The laser therapy device 10 according to the present embodiment may perform laser therapy. For example, the laser therapy device 10 may perform low-level laser therapy.

When power is supplied to the laser therapy device 10 at the extracorporeal end, a medical high-frequency current may be directly supplied to the laser therapy device 10, and to this end, in an embodiment, the laser therapy device 10 may further include a configuration capable of changing a variable resistance at a portion thereof at the extracorporeal end. For example, as the variable resistance of the portion of the laser therapy device 10 at the extracorporeal end is increased, the high-frequency current may be converted into a current of a magnitude suitable for low-level laser therapy.

In an optional embodiment, the laser therapy device 10 may further include a rechargeable battery at the other end thereof, and the rechargeable battery may be rapidly charged using power 300 energy supplied from the above-described battery, for example, a medical high-frequency generator.

In an embodiment, the laser therapy device 10 may stay in a standby mode until receiving appropriate power, and may be switched to a treatment mode by an operator upon receiving the appropriate power. To this end, in an optional embodiment, the laser therapy device 10 may include a switch for switching the standby mode to the treatment mode. For example, the switch may be a pedal, a button, or a rotary switch. However, the present disclosure is not limited thereto.

Laser irradiation in the laser therapy device 10 may be performed in a pulsed mode or a continuous irradiation mode. For example, light irradiated from the laser therapy device 10 may be light with a variation in intensity so as to have a period, light that is periodically irradiated and stopped, or light that is continuously irradiated.

In an optional embodiment, the laser therapy device 10 may further include a timer for controlling a duration of the laser therapy.

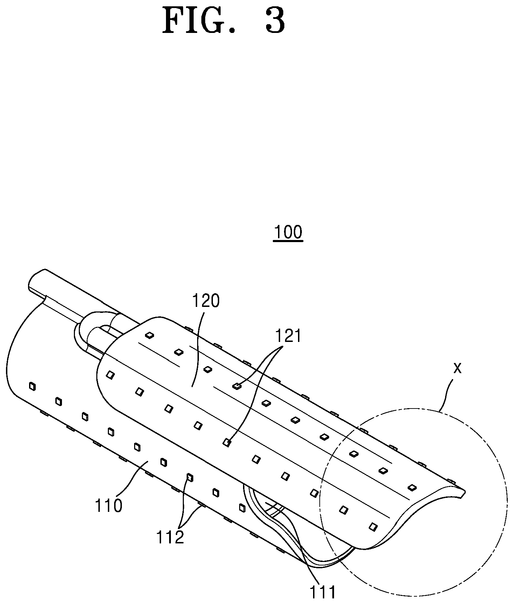

FIG. 3 is a view schematically illustrating an embodiment of the body 100 of FIG. 2.

Referring to FIG. 3, the body 100 may include the endoscope cap 110 and a hood 120.

In an embodiment, the laser therapy device may include the catheter 200 formed to extend to be inserted into the patient's body and provided with an air insufflation part, and the endoscope cap 110 having the through hole 111 through which the catheter 200 is inserted, the hood 120 disposed in one region outside the endoscope cap 110 and disposed such that at least one region thereof protrudes forward from the endoscope cap 110, and one or more LEDs disposed on each of the endoscope cap 110 and the hood 120.

As described above, the catheter 200 may be a component inserted into the patient's body.

In an embodiment, the catheter 200 may include the air insufflation part. The air insufflation part may be a component for stretching duodenal mucosal folds inside the patient's duodenum via air insufflation, for example, may be a component for stretching duodenal mucosal folds at the portion with which the laser therapy device 10 comes into contact. As a result, a large area of duodenal mucosa that has been stretched using the air insufflation part may be irradiated with light, leading to an enhancement in light irradiation efficiency.

In other words, the air insufflation part may enhance the effectiveness of a light source by stretching the duodenal mucosal folds, and specifically, when the light source used for light irradiation is a surface light source, the effectiveness of the surface light source may be maximized.

The endoscope cap 110 may be a component for the catheter 200 to be inserted.

In an embodiment, the endoscope cap 110 may include the through hole 111 provided to pass through the inside thereof to allow the catheter 200 to be inserted through the through hole 111. For example, the through hole 111 may be formed inside the endoscope cap 110 to have a length to allow the catheter 200 to be inserted. Thus, the catheter 200 may be inserted a certain length into the through hole 111 and securely fixed.

As such, the laser therapy device according to the present disclosure has a structure in which the through hole 111 is formed in the endoscope cap 110 such that the catheter 200 is insertable into the endoscope cap 110, and thus may eventually be formed in a modular structure. For example, the operator may select whether to use the body 100 in combination with the catheter 200 depending on the purpose of the treatment.

An outer surface of the endoscope cap 110 may be formed as a curved surface, for example, a substantially cylindrical shape. Thus, since there is no protruding portion to be angled, it is possible to reduce a risk of damaging the mucosa of the organ of a patient, such as the duodenal mucosa, when the endoscope cap 110 is inserted into the patient's body.

In addition, since the outer surface of the endoscope cap 110 is formed as a curved surface, as will be described below, first LEDs 112 may be radially disposed along the surface of the endoscope cap 110, and a first light L1 may be irradiated to a large area of the lower mucosa of the duodenum. In consideration of such a curved surface structure and the bends and mucosal folds of the duodenum, as will be described below, a light source, for example, a surface light source using an organic light-emitting diode (OLED), may be disposed in the curved hood 120.

In addition, the endoscope cap 110 may be formed of a transparent material. For example, the endoscope cap 110 may be a transparent cap.

The hood 120 may be a component disposed in one region outside the endoscope cap 110. For example, the hood 120 may be coupled to the outside of the endoscope cap 110 and may be formed such that at least one region thereof protrudes forward from the endoscope cap 110.

In an embodiment, the hood 120 may be disposed on an upper side of the endoscope cap 110 with respect to FIG. 3. Accordingly, as will be described below, when the body 100 is inserted into the patient's duodenum, second LEDs 121 disposed on the hood 120 may irradiate a second light L2 from inside the duodenum toward an upper side of the duodenum, for example, the upper mucosa of the duodenum and the pancreas.

At this point, since the hood 120 is disposed on the upper side of the endoscope cap 110, the first LEDs 112 may be disposed on a lower side surface of the endoscope cap 110. Thus, when the body 100 is inserted into the patient's duodenum, the first LEDs 112 disposed in the endoscope cap 110 may irradiate the first light L1 from inside the duodenum toward a lower side of the duodenum, for example, the lower mucosa of the duodenum.

Thus, when the body 100 is inserted into the patient's duodenum, different lights may be irradiated in upward and downward directions, respectively, and as necessary, lights having different wavelength bands may be irradiated. That is, it is possible to simultaneously treat the upper and lower parts of the patient's body with respect to the body 100 in a state in which a single device is inserted into the patient's body. In addition, by arranging LEDs irradiating lights of different wavelength bands on the upper and lower sides of the body 100, therapies having different effects may be performed. In addition, a quantum dot light source may be used to switch to a desired optical wavelength. This will be discussed in more detail later.

The hood 120 may be formed to have a curved surface having a curvature. For example, the hood may be formed in the shape of a plate-like wing formed to protrude toward an upper surface of the body 100. Thus, in a state in which the hood 120 is coupled to the endoscope cap 110, the overall shape of the body 100 may be formed as a substantially column shape. Accordingly, even when the body 100 enters the interior of the patient's body, the risk of damaging the mucous membranes of the duodenum or other organs may be reduced.

The hood 120 is formed as a joint structure. For example, the hood 120 may be configured to include at least one joint. By including at least one joint, the hood 120 may be flexible, thereby minimizing damage to the surrounding mucous membranes when inserted into the patient's duodenum.

For example, the hood 120 may include a plurality of joints, and may be flexible in both a longitudinal direction and a direction perpendicular thereto. That is, the hood 120 may be bent to be curved in the longitudinal direction, and may be bent to be curved in a width direction.

In addition, as will be described below, the hood 120 is formed as a joint structure so that at least one region may be lifted in a direction in which the pancreas is located, and may be bent to be curved, for example, in the longitudinal direction and lifted.

The hood 120 may be formed with a length of 4 cm to 6 cm so as to be easily inserted into the patient's duodenum to perform therapy. When the hood 120 is formed with a length of less than 4 cm, the second LEDs 121 are not sufficiently disposed on the surface of the hood 120, and thus an irradiation range of the second light L2 may be reduced. In addition, when the hood 120 is formed with a length greater than 6 cm, the hood 120 may not be easy to enter the patient's duodenum, and the risk of damaging the surrounding mucosa during entry may be increased.

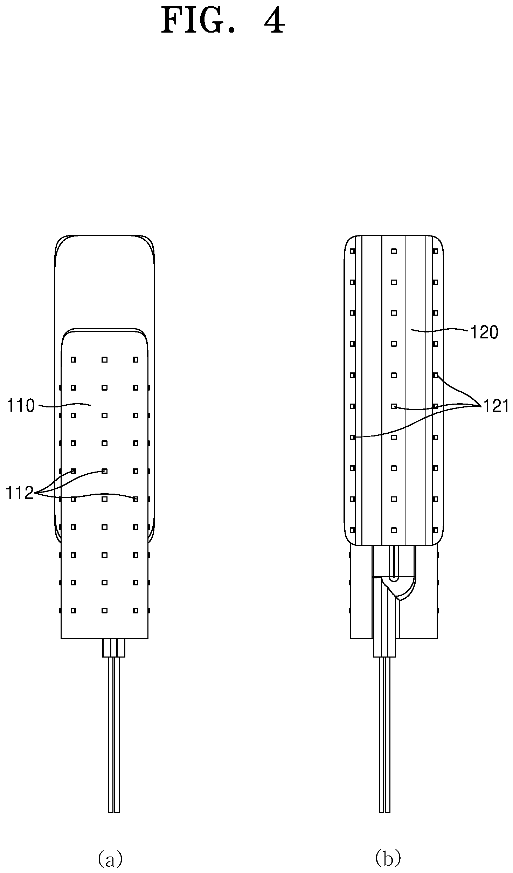

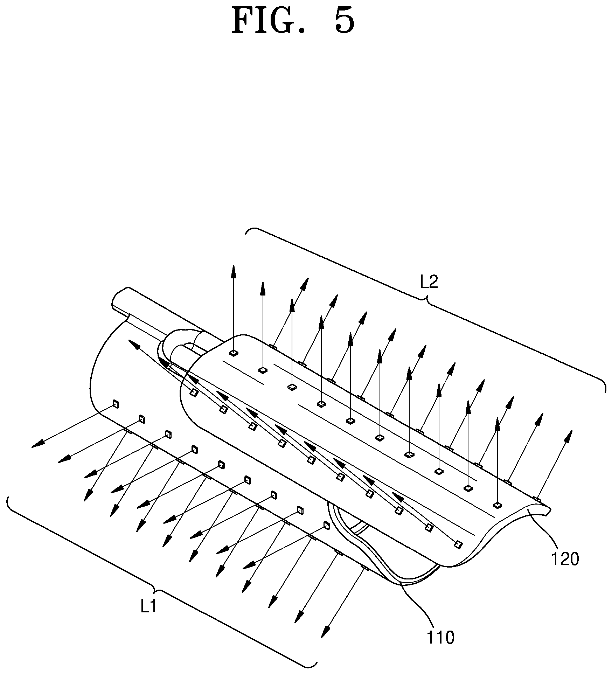

FIG. 4A is a bottom view of the body 100 of FIG. 2, FIG. 4B is a top view of the body 100 of FIG. 2, and FIG. 5 is a view illustrating light emitted by the LEDs.

Referring to FIG. 4A, one or more first LEDs 112 may be disposed in the endoscope cap 110. For example, one or more first LEDs 112 may be disposed on a lower surface of the endoscope cap 110.

The first LED 112 may be a component for irradiating the first light L1 of a first wavelength band. For example, the first LED 112 may be a point light source so as to be optimized for the curved surface structure of the endoscope cap 110, and specifically, the first LED 112 may be a mini LED, a micro LED, or an OLED. Accordingly, the endoscope cap 110 including the first LED 112 may be miniaturized, and an appropriate treatment may be performed inside the patient's body, and in an optional embodiment, the first LED 112 may be a quantum dot LED using quantum dots as a light source. Thus, by adjusting only the size of particles, the frequency and wavelength of the first light L1 irradiated through the first LED 112 may be efficiently controlled, and power consumption may be reduced. That is, when the first LED 112 is a quantum dot LED, an optical wavelength conversion may be relatively free.

A plurality of first LEDs 112 is disposed on the surface of the endoscope cap 110 and the first LEDs 112 are disposed to form a plurality of columns and plurality of rows. For example, the first LEDs 112 may be radially disposed on the surface of the endoscope cap 110.

Accordingly, as shown in FIG. 5, the first light L1 irradiated from the first LEDs 112 is not only irradiated in a linear direction from the endoscope cap 110, but may also be irradiated radially to be irradiated to a large area of the lower mucosa of the duodenum.

In another embodiment, the first LED 112 may be a flexible LED, for example, a flexible OLED. That is, the first LED 112 may be a light source using an OLED disposed on the surface of the endoscope cap 110. Accordingly, the first LED 112 may be easily disposed on the surface of the endoscope cap 110, and the first LED 112 may be disposed to form a curved surface so as to correspond to the shape of the surface of the endoscope cap 110, so that the first light L1 may be efficiently irradiated to the lower mucosa of the duodenum.

In another optional embodiment, the first LEDs 112 may be a quantum dot LED and a flexible OLED disposed independently for each wavelength. For example, the quantum dot LED and the flexible OLED may be independently disposed on the surface of the endoscope cap 110 for each wavelength. Thus, the first LEDs 112 may irradiate light of a desired wavelength to a desired part of the duodenum, thereby improving therapy efficiency.

In another optional embodiment, the first LEDs 112 may be disposed in a hybrid form in which a quantum dot LED and a flexible OLED are mixed. Accordingly, the first LEDs 112 may efficiently irradiate the first light L1 to the lower mucosa of the duodenum, thereby improving therapy efficiency.

Referring to FIG. 4B, one or more second LEDs 121 may be disposed in the hood 120. For example, one or more second LEDs 121 may be disposed on an upper surface of the hood 120.

The second LED 121 may be a component for irradiating the second light L2 of a second wavelength band. For example, the second LED 121 may be a point light source so as to be optimized for the curved surface structure of the hood 120, and specifically, the second LED 121 may be a mini LED, a micro LED, or an OLED. Thus, the hood 120 including the second LED 121 may be miniaturized and may perform an appropriate treatment inside the patient's body.

In an optional embodiment, the second LED 121 may be a quantum dot LED using quantum dots as a light source. Thus, by adjusting only the size of particles, the frequency and wavelength of the second light L2 irradiated through the second LED 121 may be efficiently controlled, and power consumption may be reduced. That is, when the second LED 121 is a quantum dot LED, an optical wavelength conversion may be relatively free.

A plurality of second LEDs 121 is disposed on the surface of the hood and the second LEDs 121 are disposed to form a plurality of columns and plurality of rows. For example, the second LEDs 121 may be radially disposed on the surface of the hood 120.

Accordingly, as shown in FIG. 5, the second light L2 irradiated from the second LED 121 may not be irradiated only in a linear direction from the hood 120, but may also be irradiated radially to be irradiated to a large area of the upper mucosa of the duodenum, and further, the second light L2 may be irradiated to the pancreas.

In another embodiment, the second LED 121 may be a flexible LED, for example a flexible OLED. That is, the second LED 121 may be a surface light source using an OLED disposed on the surface of the hood 120. Accordingly, the second LED 121 may be easily disposed on the surface of the hood 120, and the second LED 121 may be disposed to form a curved surface so as to correspond to the shape of the surface of the hood 120, so that the second light L2 may be efficiently irradiated to the upper mucosa of the duodenum.

In another optional embodiment, the second LEDs 121 may be a quantum dot LED and a flexible OLED disposed independently for each wavelength. For example, the quantum dot LED and the flexible OLED may be independently disposed on the surface of the hood 120 for each wavelength. Thus, the second LEDs 121 may irradiate light of a desired wavelength to a desired part of the duodenum, thereby improving therapy efficiency.

In another optional embodiment, the second LEDs 121 may be disposed in a hybrid form in which a quantum dot LED and a flexible OLED are mixed. Accordingly, the second LED 121 may efficiently irradiate the second light L2 to the upper mucosa of the duodenum, thereby improve therapy efficiency.

Referring to FIG. 5, the first LEDs 112 disposed in the endoscope cap 110 may irradiate the first light L1, and the second LEDs 121 disposed in the hood 120 may irradiate the second light L2.

As described above, the first LED 112 and the second LED 121 are disposed in opposite directions to each other and irradiate the lights in the downward and upward directions, respectively. Accordingly, lights having different wavelength bands may be irradiated in different directions.

The first LEDs 112 for irradiating the first light L1 of the first wavelength band may be disposed on the surface of the endoscope cap 110, and the second LEDs 121 for irradiating the second light L2 of the second wavelength band different from the first wavelength band may be disposed on the surface of the hood 120.

The first light L1 and the second light L2 may be lights irradiated for cell regeneration therapy of the duodenal mucosa or pancreas.

In an embodiment, the first wavelength band may be a predetermined wavelength band between 600 nm and 700 nm, and the second wavelength band may be a predetermined wavelength band between 800 nm and 900 nm.

The first light L1 is light having a wavelength band of the first wavelength band and irradiated toward the lower mucosa of the duodenum, and may exhibit a relatively high surface temperature. Accordingly, by irradiating the first light L1, the effect of photodynamic therapy as well as efficient photothermal therapy may be expected at the same time.

The second light L2 is light having a wavelength band of the second wavelength band and irradiated toward the upper mucosa of the duodenum, and may exhibit high transmission power relative to energy efficiency. Thus, when the second light L2 is irradiated toward the upper mucosa of the duodenum, the second light L2 may reach the pancreas located at an upper side of the duodenum, enabling cell regeneration therapy, i.e., wound healing.

In an optional embodiment, a distribution ratio of the first wavelength band and the second wavelength band may be 2:1 or 3:1. For example, the distribution ratio of the first light L1 and the second light L2 may be 2:1 or 3:1. Through this, the therapy efficiency of the laser therapy device 10 may be further improved.

According to the present disclosure, efficient therapy can be achieved by irradiating lights having a plurality of wavelength bands in different directions using a single device. For example, by irradiating the first light L1 toward the lower mucosa of the duodenum and irradiating the second light L2 toward the upper mucosa of the duodenum, it is possible to promote the regeneration of duodenal and pancreatic cells, and suppress the function of cells involved in secreting diabetes-related hormone such as GIP and aid in the transformation of these cells into normal cells. In other words, by irradiating light on the surface of the duodenum via LEDs, it is possible to improve the function of mitochondria of normal cells, inhibit the function of K cells by increasing ATP production, and trigger the regeneration of normal duodenal and pancreatic cells. In addition, according to the present disclosure, type 2 diabetes and metabolic diseases may be treated by improving the function of normal resident flora in the small intestine and suppressing the proliferation of abnormal resident flora related to diabetes and metabolic diseases through efficient photo microbiota transplantation (PMT).

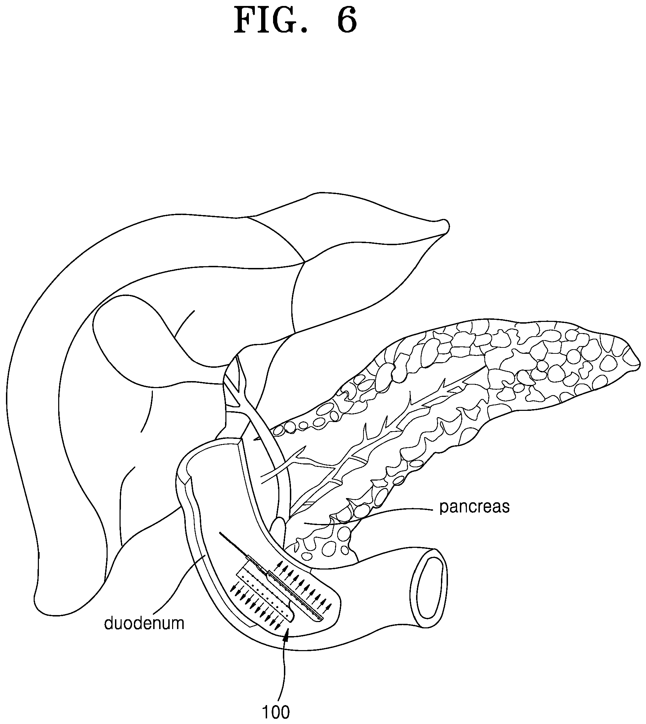

FIG. 6 is a view for describing an example of use of the low-level laser therapy device 10 using an LED endoscope cap according to the present disclosure.

Referring to FIG. 6, the body 100 may be inserted into the duodenum, and may irradiate light to the upper mucosa of the duodenum and the pancreas, and the lower mucosa of the duodenum.

In an optional embodiment, when the body 100 is inserted into the patient's duodenum and the hood 120 comes in contact with the upper mucosa of the duodenum, the amount of air insufflation from the air insufflation part may be reduced. For example, the operator may reduce the amount of air insufflation through the air insufflation part by bringing the hood 120 into contact with the desired part. At this time, as the amount of air insufflation is reduced, the lumen of the duodenum may contract, so that the upper mucosa of the duodenum may be in contact with the hood 120 over a larger area. In addition, as described above, since the hood 120 is formed in a joint structure and has flexibility, the hood 120 may be in contact with the upper mucosa of the duodenum over a larger area when the amount of air insufflation is reduced. In addition, in an embodiment, the hood 120 may be configured with a transparent cap structure so that the degree of close contact with the duodenal mucosa may be identified in real time.

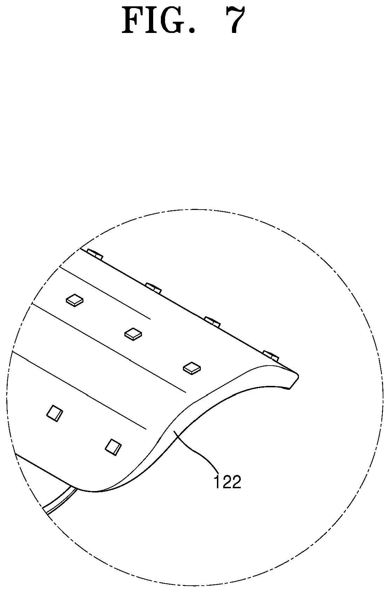

FIG. 7 is an enlarged view of portion x of FIG. 3.

Referring to FIG. 7, the hood 120 may include a curved region 122, which is formed as a curved surface, at one end thereof.

The body 100 is connected to the catheter 200 and inserted into the patient's body. Thus, as the body 100 is inserted through the interior of the human body, a problem of damaging the mucosa or surface of the organ may arise. Accordingly, in order to minimize such damage, the components constituting the body 100 may each be formed with a curved surface.

The curved region 122 may be formed at one end of the hood 120, for example, at the foremost end portion of the hood 120.

The curved region 122 is located at the foremost end portion of the hood 120 and may be formed with a curved and smooth surface, and thus may be smoothly slid when coming into contact with the mucous membranes of the duodenum or other internal organs.

Accordingly, due to the nature that the foremost end portion of the hood 120, which is located at the foremost end of the body 100, is the first and most likely point of contact with the surrounding mucosa as the body 100 enters the patient's duodenum, by forming the curved region 122 at the foremost end portion of the hood 120, damage to the mucosa may be minimized.

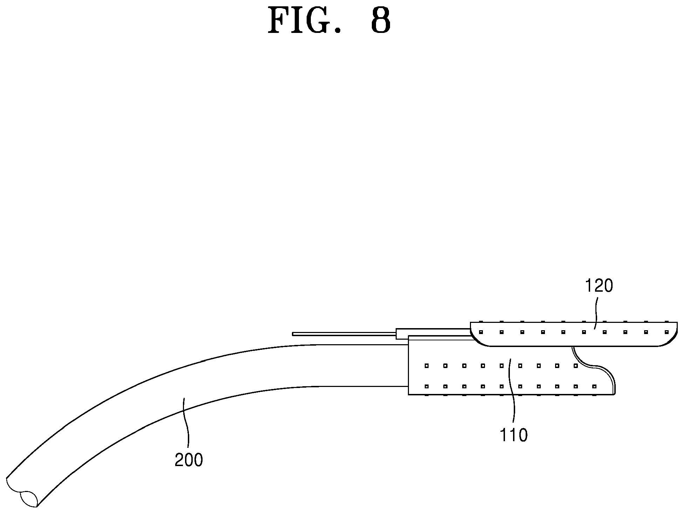

FIG. 8 is a side view of the low-level laser therapy device 10 using an LED endoscope cap of FIG. 2.

Referring to FIG. 8, the hood 120 may be disposed in one region outside the endoscope cap 110, and may be disposed such that at least one region thereof protrudes forward from the endoscope cap 110. Here, the term "forward" means the longitudinal direction of the hood 120, means a direction opposite the portion of the endoscope cap 110 to which the catheter 200 is connected, and is the right side with respect to FIG. 8.

Referring to FIG. 6 again, in consideration of a direction in which the body 100 is inserted into the lumen of the duodenum, in order to irradiate light to the upper mucosa of the duodenum and the pancreas, it is preferable to irradiate a laser at a position deeper than the lower mucosa. However, to this end, when the body 100 is inserted to a deeper position, a problem arises that the lower mucosa of the duodenum is not sufficiently irradiated by the laser.

In order to address this problem, the hood 120 and the endoscope cap 110 may be positioned differently relative to each other, for example, at least one region of the hood 120 may be located to protrude forward from the endoscope cap 110.

Accordingly, the second LED 121, which is disposed in the hood 120, may be positioned relatively deeper in the duodenum compared to the first LED 112 disposed in the endoscope cap 110. Thus, the second LED 121 may irradiate the second light L2 to a large area of the upper mucosa of the duodenum and the pancreas, and the first LED 112 may irradiate the first light L1 to a large area of the lower mucosa of the duodenum.

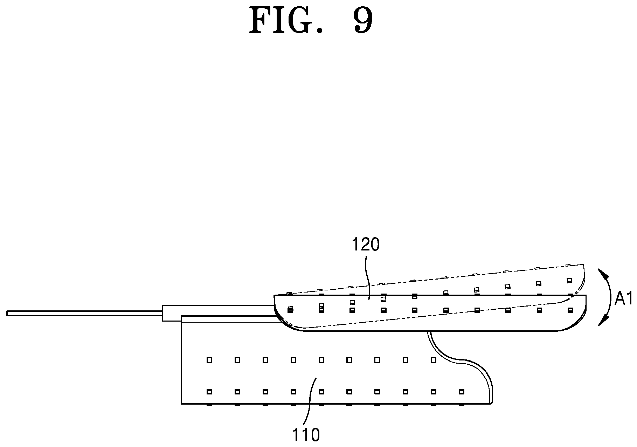

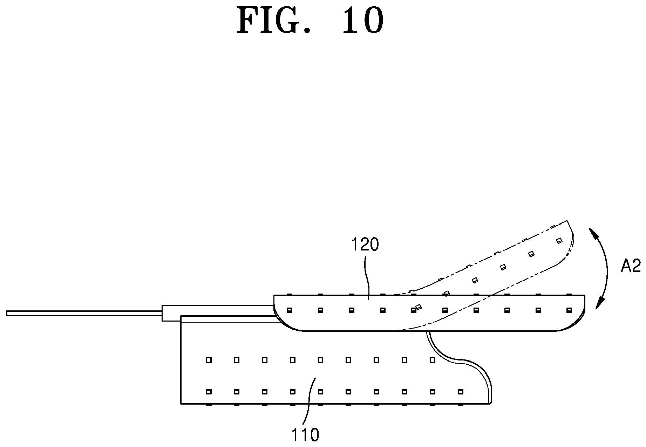

FIG. 9 is a view illustrating an embodiment of the hood 120 of FIG. 3, and FIG. 10 is a view illustrating another embodiment of the hood 120 of FIG. 3.

Referring to FIG. 6 again, since the interior of the duodenum is formed to be curved, in order to ensure maximum contact area of the hood 120 with the upper mucosa, it is preferable that the angle of the hood 120 is changed. For example, when the body 100 is disposed such that an angle is formed between the hood 120 and the endoscope cap 110 when the body 100 is located inside the duodenum, the LED disposed in the hood 120 may come into contact with a large area of the upper mucosa, and the LED disposed in the endoscope cap 110 may still face the lower mucosa of the duodenum.

Accordingly, to this end, the hood 120 is movable such that at least one region thereof forms a predetermined angle with respect to the endoscope cap 110.

Referring to FIG. 9, the hood 120 may be rotatable in a state in which one side thereof is coupled and fixed to the endoscope cap 110. For example, the hood 120 may be hingedly coupled to the endoscope cap 110. In this case, the hinge-coupled portion may be a rotation axis of the hood 120.

In an embodiment, a rotation angle A1 of the hood 120 may range from 5° to 15°. When the rotation angle of the hood 120 is less than or equal to 5°, a problem may arise that the LED disposed in the hood 120 does not come into contact with more than an appropriate area of the upper mucosa. In addition, when the rotation angle of the hood 120 is greater than or equal to 15°, a problem may arise that an area in which the upper mucosa and the hood 120 come in contact with each other may be reduced.

Referring to FIG. 10, the hood 120 may be bent such that a partial region in front of the hood 120 is curved. For example, a portion of the hood 120 that is coupled to the endoscope cap 110 may remain unbent, while the other side may be bent towards the upper mucosa.

In an embodiment, an angle at which a front portion of the hood 120 is bent may range from 5° to 15°. When the front portion of the hood 120 is bent at an angle of 5° or less, the LED disposed in the hood 120 does not come into contact with more than the appropriate area of the upper mucosa. In addition, when the front portion of the hood 120 is bent at an angle of 15° or more, a problem may arise that an area in which the upper mucosa and the hood 120 come in contact with each other may be reduced.

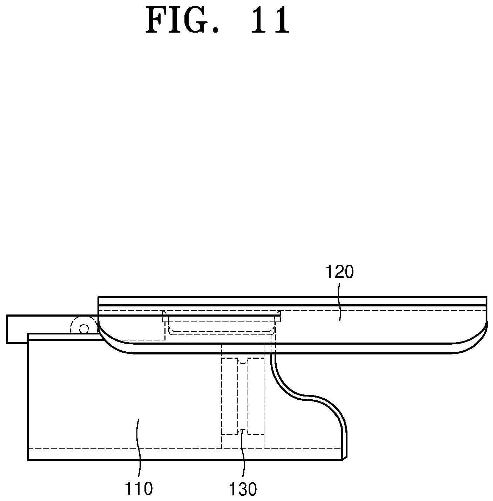

FIG. 11 is a view for describing the interior of the body 100.

Referring to FIG. 11, the laser therapy device according to an embodiment of the present disclosure may further include a band 130.

For example, the band 130 having a diameter less than a diameter of the through hole 111 may be inserted into the through hole 111.

In general, since a tube used for an endoscope varies in size, it is preferable that various tubes are inserted and fixed as necessary. Accordingly, when the catheter 200 having a diameter less than the diameter of the through hole 111 is inserted, in order to securely fix the catheter 200, the band 130 having a diameter less than the diameter of the through hole 111 may be inserted into the through hole 111 That is, as the band 130 is inserted into the through hole 111, the laser therapy device 10 according to the present embodiment may be compatible with endoscopes having various diameters.

In an embodiment, in order to easily insert the catheter 200 and securely fix the inserted catheter 200, the band 130 may be formed of a flexible material. For example, the band 130 may be formed of a silicon material.

In an embodiment, the band 130 may be formed as a ring shape. For example, the band 130 may be formed as a ring shape with a circular hole formed therein. However, the present disclosure is not limited thereto, and any structure capable of easily fixing the catheter 200 inserted into the through hole 111 may be employed.

In an optional embodiment, the band 130 may be detachably inserted into the through hole 111 formed in the endoscope cap 110. For example, when the operator uses the catheter 200 having a relatively small diameter, the band 130 may be inserted into the through hole 111 to ensure that the catheter 200 is securely inserted or fixed. On the contrary, when the operator uses the catheter 200 having a relatively large diameter, the catheter 200 may be directly inserted into the through hole 111 in a state in which the band 130 is not inserted into the through hole 111, and used.

The present disclosure has been described with reference to the examples illustrated in the drawings, but these are only examples. It will be understood by those skilled in the art that various modifications may be made. Accordingly, the true technical scope of the present disclosure is defined by the appended claims.

The particular implementations shown and described herein are illustrative examples of the embodiments and are not intended to otherwise limit the scope of the embodiments in any way. For the sake of brevity, conventional electronics, control systems, software development and other functional aspects of the systems may not be described in detail. Further, the connecting lines or connectors shown in the drawings are intended to represent example functional relationships and/or physical or logical couplings between the various elements. It should be noted that many alternative or additional functional relationships, physical connections, or logical connections may be present in a practical device. In addition, no item or element is essential to the practice of the present disclosure unless the element is specifically described as "essential" or "critical".

The use of the terms "a" and "an" and "the" and similar referents in the context of describing the present disclosure (especially in the context of the following claims) are to be construed to cover both the singular and the plural. Further, recitation of ranges of values herein are merely intended to serve as a shorthand method of referring individually to each separate value falling within the range, unless otherwise indicated herein, and each separate value is incorporated into the specification as if it were individually recited herein. Finally, operations of all methods described herein can be performed in any suitable order unless otherwise indicated herein or otherwise clearly contradicted by context. The present disclosure is not necessarily limited to the described order of the operations. The use of any and all examples, or exemplary terms (e.g., "such as") provided herein, is intended merely to better illuminate the present disclosure and does not pose a limitation on the scope of the present disclosure unless otherwise claimed. The invention is defined by the appended set of claims.

### INDUSTRIAL APPLICABILITY

According to an embodiment of the present disclosure, a low-level laser therapy device using an LED endoscope cap is provided. In addition, the embodiment of the present disclosure may be applied to a low-level laser therapy device used in industry.

## Claims

1. A low-level laser therapy device using a light-emitting diode, LED, endoscope cap, the low-level laser therapy device comprising:
a catheter (200) which is formed to extend so as to be inserted into a body of a patient and includes an air insufflation part for stretching the mucosal folds of the duodenum of the patient;
an endoscope cap (110) having a through hole (111) therein to allow the catheter (200) to be inserted therethrough;
a hood (120) disposed in one region outside the endoscope cap (110) and disposed such that at least one region thereof protrudes forward from the endoscope cap(110); and
a plurality of LEDs disposed on each of the endoscope cap (110) and the hood (120),
wherein a plurality of first LEDs (112) is disposed on a surface of the endoscope cap (110) to form a plurality of columns and plurality of rows,
wherein a plurality of second LEDs (121) is disposed on a surface of the hood (120) to form a plurality of columns and plurality of rows,
wherein the hood (120) is formed to have a joint structure and is movable such that at least one region thereof forms a predetermined angle with respect to the endoscope cap (110).

2. The low-level laser therapy device of claim 1, wherein the hood (120) includes a curved region (122) having a curved surface at one end thereof.

3. The low-level laser therapy device of claim 1, wherein
the plurality of first LEDs (112) is configured to irradiate a first light of a first wavelength band, and
the plurality of second LEDs (121) is configured to irradiate a second light of a second wavelength band.

4. The low-level laser therapy device of claim 3, wherein
the first wavelength band is a predetermined wavelength band between 600 nm and 700 nm, and
the second wavelength band is a predetermined wavelength band between 800 nm and 900 nm.

5. The low-level laser therapy device of claim 1, wherein the LEDs include at least one of a quantum dot LED with quantum dots as a light source, and a flexible organic light-emitting diode ,OLED.

## Patentansprüche

1. Low-level-Lasertherapievorrichtung unter Verwendung einer Endoskopkappe mit Leuchtdioden, LEDs, wobei die Low-level-Lasertherapievorrichtung Folgendes umfasst:
einen Katheter (200), der so ausgebildet ist, dass er sich so erstreckt, dass er in einen Körper eines Patienten geschoben wird, und einen Lufteinblasteil zum Dehnen der Schleimhautfalten des Zwölffingerdarms des Patienten aufweist;
eine Endoskopkappe (110) mit einer Durchgangsbohrung (111) darin, damit der Katheter (200) dort hindurch eingeschoben werden kann;
eine Abdeckung (120), die in einem Bereich außerhalb der Endoskopkappe (110) angeordnet ist und derart angeordnet ist, dass mindestens ein Bereich davon aus der Endoskopkappe (110) nach vorn ragt; und
eine Vielzahl von LEDs, die sowohl an der Endoskopkappe (110) als auch der Abdeckung (120) angeordnet sind,
wobei eine Vielzahl erster LEDs (112) an einer Fläche der Endoskopkappe (110) angeordnet ist und eine Vielzahl von waagerechten Reihen und eine Vielzahl von senkrechten Reihen bildet,
wobei eine Vielzahl zweiter LEDs (121) an einer Fläche der Abdeckung (120) angeordnet ist und eine Vielzahl von waagerechten Reihen und eine Vielzahl von senkrechten Reihen bildet,
wobei die Abdeckung (120) so ausgebildet ist, dass sie einen Gelenkaufbau aufweist und derart beweglich ist, dass mindestens ein Bereich davon bezogen auf die Endoskopkappe (110) einen vorher festgelegten Winkel bildet.

2. Low-level-Lasertherapievorrichtung nach Anspruch 1, wobei die Abdeckung (120) einen gekrümmten Bereich (122) aufweist, der an einem Ende davon eine gekrümmte Fläche aufweist.

3. Low-level-Lasertherapievorrichtung nach Anspruch 1, wobei
die Vielzahl erster LEDs (112) so ausgelegt ist, dass sie ein erstes Licht in einem ersten Wellenlängenband ausstrahlt, und
die Vielzahl zweiter LEDs (121) so ausgelegt ist, dass sie ein zweites Licht in einem zweiten Wellenlängenband ausstrahlt.

4. Low-level-Lasertherapievorrichtung nach Anspruch 3, wobei
das erste Wellenlängenband ein vorher festgelegtes Wellenlängenband zwischen 600 nm und 700 nm ist, und
das zweite Wellenlängenband ein vorher festgelegtes Wellenlängenband zwischen 800 nm und 900 nm ist.

5. Low-level-Lasertherapievorrichtung nach Anspruch 1, wobei die LEDs eine Quantenpunkt-LED mit Quantenpunkten als Lichtquelle und/oder eine flexible organische Leuchtdiode, OLED, umfassen.

## Revendications

1. Dispositif de thérapie laser de faible niveau utilisant une diode luminescente, LED, un capuchon d'endoscope, le dispositif de thérapie laser de faible niveau comprenant
un cathéter (200) qui est formé pour s'étendre de manière à être inséré dans un corps d'un patient et comprend une partie d'insufflation d'air pour étirer les replis mucosals du duodénum du patient ;
un capuchon d'endoscope (110) ayant un trou (111) à l'intérieur pour permettre au cathéter (200) d'être inséré à travers celui-ci,
une capuche (120) disposée dans une zone à l'extérieur du capuchon d'endoscope (110) et disposée de telle manière qu'au moins une de ses zones fait saillie vers l'avant du capuchon d'endoscope (110) ; et
une pluralité de LED disposés sur chacun du capuchon d'endoscope (110) et de la capuche (120),
une pluralité de premiers LED (112) étant disposés sur une surface du capuchon d'endoscope (110) pour former une pluralité de colonnes et une pluralité de rangées,
une pluralité de seconds LED (121) étant disposés sur une surface de la capuche (120) pour former une pluralité de colonnes et une pluralité de rangées,
la capuche (120) étant formée pour avoir une structure de joint et étant amovible de telle manière qu'au moins une de ses zones forme un angle prédéterminé par rapport au capuchon d'endoscope (110).

2. Dispositif de thérapie laser de faible niveau selon la revendication 1, dans lequel la capuche (120) comprend une zone incurvée (122) ayant une surface incurvée à une de ses extrémités.

3. Dispositif de thérapie laser de faible niveau selon la revendication 1, dans lequel
la pluralité de premiers LED (112) est configurée pour irradier une première lumière d'une première bande de longueur d'onde et
la pluralité de seconds LED (121) est configurée pour irradier une seconde lumière d'une seconde bande de longueur d'onde.

4. Dispositif de thérapie laser de faible niveau selon la revendication 3, dans lequel
la première bande de longueur d'onde est une bande de longueur d'onde prédéterminée comprise entre 600 nm et 700 nm et
la seconde bande de longueur d'onde est une bande de longueur d'onde prédéterminée comprise entre 800 nm et 900 nm.

5. Dispositif de thérapie laser de faible niveau selon la revendication 1, dans lequel les LED comprennent au moins un de LED à point quantique avec des points quantiques comme source de lumière et une diode luminescente organique flexible (OLED).
